# EUROPEAN PATENT APPLICATION

(11) **EP 3 951 797 A1**
(43) Date of publication of application: **09.02.2022**
(21) Application number: 20808831.0
(22) Date of filing: 15.05.2020
(51) Int. Cl.: G16H 50/30, A61B 5/00

(54) **HEAT-STRESS EFFECT ESTIMATING DEVICE, HEAT-STRESS EFFECT ESTIMATING METHOD, AND COMPUTER PROGRAM**

(30) Priority: 17.05.2019 JP 2019093363
(71) Applicant: DAIKIN INDUSTRIES, LTD., Osaka-shi, Osaka 530-8323 (JP); RIKEN, Wako-shi Saitama 351-0198 (JP)
(72) Inventor: SAKATA Youko, Osaka-shi, Osaka 530-8323 (JP); HORI Shouta, Osaka-shi, Osaka 530-8323 (JP); HASHIMOTO Satoshi, Osaka-shi, Osaka 530-8323 (JP); WATANABE Yasuyoshi, Wako-shi, Saitama 351-0198 (JP); MIZUNO Kei, Wako-shi, Saitama 351-0198 (JP); MORITO Yusuke, Wako-shi, Saitama 351-0198 (JP); YAMANO Emi, Wako-shi, Saitama 351-0198 (JP); IWASAKI Miho, Wako-shi, Saitama 351-0198 (JP)
(74) Representative: Goddar, Heinz J.
(86) International application number: PCT/JP2020/019558
(87) International publication number: WO 2020/235509

(57) **Abstract**

A heat-stress effect estimating device (10) includes a storage unit (11) and a calculation unit (12). The storage unit (11) stores a correlation of at least an ambient temperature of a person and an outdoor air temperature with at least one index indicating an effect of a heat stress on a person. The calculation unit (12) estimates a state of a subject in terms of the at least one index, based on the correlation stored in the storage unit (11), an ambient temperature of the subject at a predetermined time point, and a history of an outdoor air temperature up to the predetermined time point.

## Description

### Technical Field

The present disclosure relates to a heat-stress effect estimating device, a heat-stress effect estimating method, and a computer program.

### Background Art

If a person is exposed to a heat stress for a long time or frequently and the amount of accumulated heat stress increases, a probability of occurrence of heatstroke, hypothermia, insomnia resulting from a sweltering night or the like, summer fatigue, fatigue due to a temperature difference, or the like increases.

In the related art, there has been proposed a system that estimates an amount of accumulated heat stress resulting from a heat environment, a work intensity, a work pattern, and the like by using a heat index (WBGT (Wet Bulb Globe Temperature)) as an index representing the heat stress and manages the heat stress (PTL 1).

### Citation List

### Patent Literature

PTL 1: Japanese Unexamined Patent Application Publication (Translation of PCT Application) No. 2009-518106

### Summary of Invention

### Technical Problem

However, in the method disclosed in PTL 1, only part of a reaction caused in a person by a heat stress such as a likelihood of heatstroke is observed, and the other physical reactions caused by the heat stress, for example, wakefulness (awake or sleepy), fatigue, and so on are not taken into account.

It is an object of the present disclosure to enable an effect of a heat stress imposed on a subject by an ambient temperature of the subject to be broadly and easily estimated to make it easier to grasp an effect of the accumulated heat stress on health and to prevent the occurrence of a disease, for example.

### Solution to Problem

In a first aspect of the present disclosure, a heat-stress effect estimating device for estimating an effect of a heat stress imposed on a subject by an ambient temperature of the subject includes a storage unit (11) configured to store a correlation of at least an ambient temperature of a person and an outdoor air temperature with at least one index indicating an effect of a heat stress on a person, and a calculation unit (12) configured to estimate a state of the subject in terms of the at least one index, based on the correlation stored in the storage unit (11), an ambient temperature of the subject at a predetermined time point, and a history of an outdoor air temperature up to the predetermined time point.

In the first aspect, a correlation of an ambient temperature of a person and an outdoor air temperature with an index indicating an effect of a heat stress on a person is determined in advance. Based on the correlation, an ambient temperature of a subject, and a history of an outdoor air temperature, the state of the subject is estimated in terms of the index. Thus, through selection of the index, an effect of a heat stress can be easily estimated in terms of various reactions such as wakefulness and fatigue caused in a person by the heat stress.

In a second aspect of the present disclosure, in the heat-stress effect estimating device according to the first aspect, a converted temperature determined by taking into account at least one of an ambient humidity, an ambient radiant heat, an ambient air flow, a metabolic rate, and an amount of clothing of the subject is used as the ambient temperature of the subject.

In the second aspect, the converted temperature determined by taking into account at least one of the ambient humidity, the ambient radiant heat, the ambient air flow, the metabolic rate, and the amount of clothing of the subject is used as the ambient temperature of the subject. Thus, the effect of the heat stress can be estimated more accurately.

In a third aspect of the present disclosure, in the heat-stress effect estimating device according to the first or second aspect, the at least one index is at least one of a degree of wakefulness, a degree of fatigue, a degree of enthusiasm, a degree of irritation, and a degree of relaxing.

In the third aspect, the degree of wakefulness, the degree of fatigue, the degree of enthusiasm, the degree of irritation, or the degree of relaxing is used as the index indicating the effect of the heat stress on the person. Thus, the effect of the heat stress can be estimated in terms of the degree of wakefulness, the degree of fatigue, the degree of enthusiasm, the degree of irritation, or the degree of relaxing.

In a fourth aspect according to the present disclosure, in the heat-stress effect estimating device according to any one of the first to third aspects, the calculation unit (12) estimates the state over a predetermined period and calculates a cumulative frequency of the estimated state.

In the fourth aspect, an amount of heat stress accumulated over the predetermined period can be evaluated.

In a fifth aspect of the present disclosure, in the heat-stress effect estimating device according to any one of the first to fourth aspects, the calculation unit (12) classifies the estimated state in accordance with at least one of a degree of the state, a duration of the state, and an occurrence time of the state, and calculates a cumulative frequency for each classification.

In the fifth aspect, the effect of the heat stress can be evaluated from multiple perspectives.

In a sixth aspect of the present disclosure, the heat-stress effect estimating device according to any one of the first to fifth aspects further includes a correction unit (23) configured to correct the correlation stored in the storage unit (11), based on a setting input by the subject or attribute information of the subject.

In the sixth aspect, the effect of the heat stress can be estimated by taking into account a difference between individuals.

In a seventh aspect of the present disclosure, in the heat-stress effect estimating device according to any one of the first to sixth aspects, the ambient temperature of the subject is measured by a temperature sensor carried by the subject.

In the seventh aspect, even when the subject moves, the ambient temperature of the subject can be easily measured.

In an eighth aspect of the present disclosure, in the heat-stress effect estimating device according to any one of the first to sixth aspects, the ambient temperature of the subject is calculated based on temperature information measured by temperature sensors installed at a plurality of places and a movement history of the subject.

In the eighth aspect, even when the subject moves without carrying a temperature sensor, the ambient temperature of the subject can be measured.

In a ninth aspect of the present disclosure, the heat-stress effect estimating device according to any one of the first to eighth aspects further includes a display unit (17) configured to display the estimated state and/or secondary information obtained based on the state.

In the ninth aspect, the effect of the heat stress can be evaluated in an easy-to-understand manner.

In a tenth aspect of the present disclosure, in the heat-stress effect estimating device according to any one of the first to ninth aspects, the calculation unit (12) compares the estimated state with a state of a person prepared in advance in terms of the at least one index on a per-health-condition basis and estimates a health condition of the subject.

In the tenth aspect, it becomes easier to accurately grasp the effect of the heat stress on health. Thus, the effect of the heat stress on health can be made use of in prevention of the occurrence of a disease.

In an eleventh aspect of the present disclosure, a heat-stress effect estimating method for estimating an effect of a heat stress imposed on a subject by an ambient temperature of the subject includes a step A of, with a computer, determining a correlation of at least an ambient temperature of a person and an outdoor air temperature with at least one index indicating an effect of a heat stress on a person and storing the correlation in a storage unit (11); and a step B of, with the computer, estimating a state of the subject in terms of the at least one index, based on the correlation stored in the storage unit (11), an ambient temperature of the subject at a predetermined time point, and a history of an outdoor air temperature up to the predetermined time point.

In the eleventh aspect, a correlation of an ambient temperature of a person and an outdoor air temperature with an index indicating an effect of a heat stress on a person is determined in advance. Based on the correlation, an ambient temperature of a subject, and a history of an outdoor air temperature, the state of the subject is estimated in terms of the index. Thus, through selection of the index, an effect of a heat stress can be easily estimated in terms of various reactions such as wakefulness and fatigue caused in a person by the heat stress.

In a twelfth aspect of the present disclosure, a computer program causes a computer to perform the heat-stress effect estimating method according to the eleventh aspect.

In the twelfth aspect, advantages similar to those of the eleventh aspect can be obtained.

### Brief Description of Drawings

[Fig. 1] Fig. 1 is a diagram illustrating an example of a correlation of a room temperature and an outdoor air temperature with sleepiness (a degree of wakefulness), which has been found by the inventors of this application.
[Fig. 2] Fig. 2 is a diagram illustrating an example of a correlation of a room temperature and an outdoor air temperature with a degree of fatigue, which has been found by the inventors of this application.
[Fig. 3] Fig. 3 is a block configuration diagram of a heat-stress effect estimating device according to an embodiment.
[Fig. 4A] Fig. 4A is a histogram illustrating an example of frequencies of states of a subject obtained in terms of various indices by the heat-stress effect estimating device according to the embodiment.
[Fig. 4B] Fig. 4B is a histogram illustrating another example of the frequencies of the states of a subject obtained in terms of the various indices by the heat-stress effect estimating device according to the embodiment.
[Fig. 5A] Fig. 5A is a diagram illustrating an example of a result obtained by the heat-stress effect estimating device according to the embodiment by classifying, in accordance with degrees of the states, the states of the subject obtained in terms of the various indices and by calculating cumulative frequencies for the respective classifications.
[Fig. 5B] Fig. 5B is a diagram illustrating an example of a result obtained by the heat-stress effect estimating device according to the embodiment by classifying, in accordance with durations of the states, the states of the subject obtained in terms of the various indices and by calculating cumulative frequencies for the respective classifications.
[Fig. 5C] Fig. 5C is a diagram illustrating an example of a result obtained by the heat-stress effect estimating device according to the embodiment by classifying, in accordance with occurrence times of the states, the states of the subject obtained in terms of the various indices and by calculating cumulative frequencies for the respective classifications.
[Fig. 6A] Fig. 6A is a diagram illustrating an example of states of a healthy person in terms of the indices, used by the heat-stress effect estimating device according to the embodiment.
[Fig. 6B] Fig. 6B is a diagram illustrating an example of states of a person in a presymptomatic state in terms of the indices, used by the heat-stress effect estimating device according to the embodiment.
[Fig. 7] Fig. 7 is a block configuration diagram of a heat-stress effect estimating device according to a modification.
[Fig. 8] Fig. 8 is a diagram illustrating an example of a correlation of a room temperature and an outdoor air temperature with a degree of enthusiasm, which has been found by the inventors of this application.
[Fig. 9] Fig. 9 is a diagram illustrating an example of a correlation of a room temperature and an outdoor air temperature with a degree of irritation, which has been found by the inventors of this application.
[Fig. 10] Fig. 10 is a diagram illustrating an example of a correlation of a room temperature and an outdoor air temperature with a degree of relaxing, which has been found by the inventors of this application.

### Description of Embodiments

An embodiment of the present disclosure will be described below with reference to the drawings. Note that the embodiment below is a preferable example in essence and does not intend to limit the scope of the present invention and of the applications or uses thereof.

### <Heat Stress Reaction Model>

As a result of tests performed on subjects under various temperature-humidity environment conditions in winter and summer, the inventors of this application have found a temperature (hereinafter, may also be referred to as a boundary temperature of sleepiness) that serves as a boundary at which the subjects start feeling sleepiness and also have found that the boundary temperature of sleepiness varies with the season.

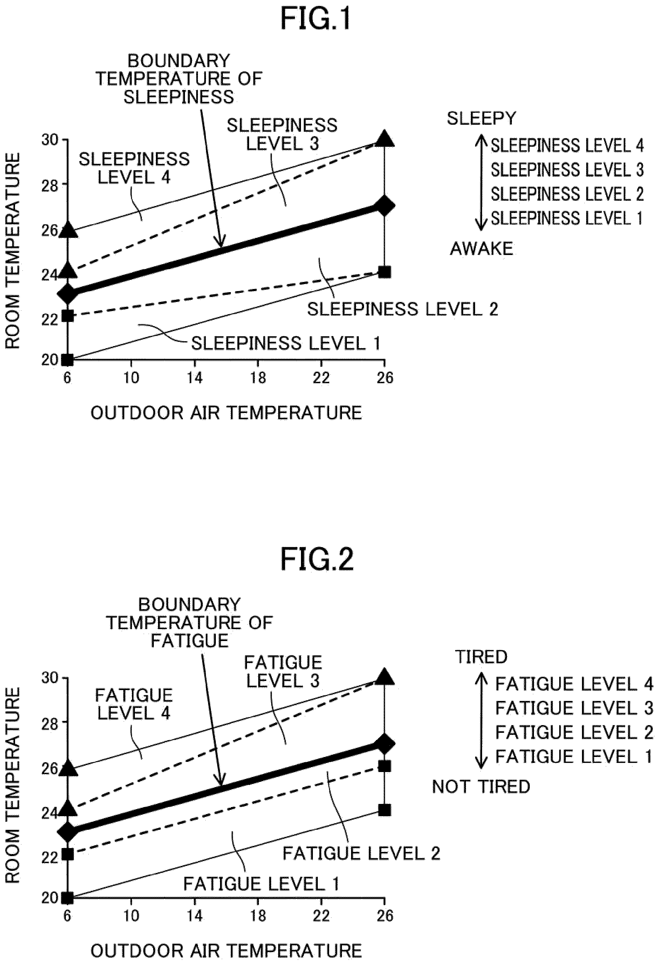

Fig. 1 is a diagram illustrating an example of a correlation (hereinafter, may also be referred to as a "heat stress reaction model") of an ambient temperature (hereinafter, may also be simply referred to as a "room temperature") of a person and an outdoor air temperature (hereinafter, may also be simply referred to as an "air temperature") with sleepiness (a degree of wakefulness) that is one of effects of a heat stress caused by the ambient temperature on the person, which has been found by the inventors of this application.

The heat stress reaction model illustrated in Fig. 1 indicates that a sleepiness-inducing room temperature is around 26°C when the air temperature is 15°C, for example. That is, if the air temperature is known, a sleepiness-inducing room temperature or an awaking room temperature can be calculated by using the heat stress reaction model illustrated in Fig. 1. Alternatively, if the room temperature and the air temperature are known, a degree of sleepiness in a room environment can be estimated by using the heat stress reaction model illustrated in Fig. 1.

Note that when the heat stress reaction model illustrated in Fig. 1 is applied, a history of the air temperature up to a target time point, for example, an average air temperature of the past week, is used as the air temperature by taking into account adaptation of the human body to the air temperature.

In addition, since the boundary temperature of sleepiness varies between individuals, a subject to which the heat stress reaction model illustrated in Fig. 1 is applied may correct the model in accordance with their preference, or the model may be corrected based on a characteristic of the subject, for example, the gender, age, metabolic rate, body fat percentage, blood pressure, or the like. The heat stress reaction model may be corrected, for example, by correcting the boundary temperature of sleepiness.

In addition, the heat stress reaction model illustrated in Fig. 1 represents a correlation of the room temperature and the air temperature with the sleepiness. However, an ambient humidity, an ambient air flow, or the like may be taken into account as an environment factor around the person in addition to the room temperature (ambient temperature). For example, a converted temperature determined by taking into account at least one of an ambient humidity, an ambient radiant heat, an ambient air flow, a metabolic rate, and an amount of clothing of the subject, for example, a sensible temperature such as SET* (standard effective temperature), may be used as the room temperature.

A model similar to the heat stress reaction model illustrated in Fig. 1 may be created by using fatigue (a degree of fatigue) or the like as the effect of the heat stress caused by the ambient temperature on the person. Fig. 2 is a diagram illustrating an example of a correlation of an ambient temperature of a person and an outdoor air temperature with a degree of fatigue which is one of the effects of the heat stress caused by the ambient temperature on a person, which has been found by the inventors of this application.

### <Configuration of Heat-Stress Effect Estimating Device>

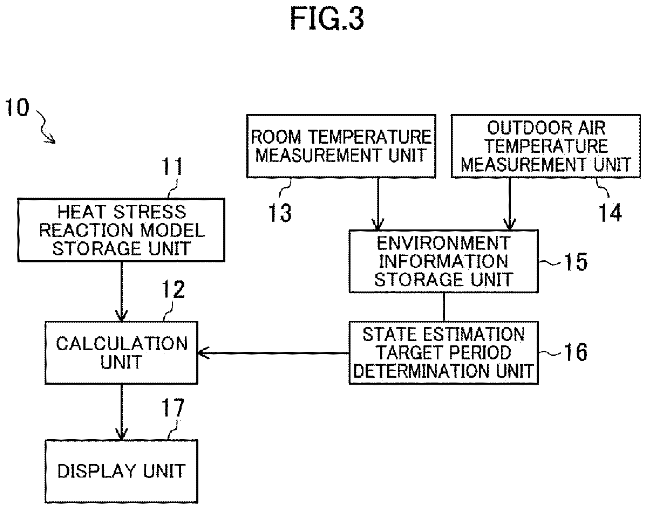

Fig. 3 is a block configuration diagram of a heat-stress effect estimating device according to an embodiment.

As illustrated in Fig. 3, a heat-stress effect estimating device (10) according to the present embodiment mainly includes a heat stress reaction model storage unit (11) and a calculation unit (12). The heat stress reaction model storage unit (11) stores a correlation of at least an ambient temperature of a person and an outdoor air temperature with at least one index indicating an effect of a heat stress on a person, for example, the heat stress reaction model illustrated in Fig. 1. The calculation unit (12) estimates a state of a subject in terms of the index, based on the correlation (heat stress reaction model) stored in the heat stress reaction model storage unit (11), an ambient temperature of the subject at a predetermined time point, and a history of an outdoor air temperature up to the predetermined time point. The index for use in the heat stress reaction model may be, for example, the degree of wakefulness, the degree of fatigue, or the like. The history of the outdoor air temperature for use in the calculation unit (12) may be, for example, an average air temperature of the past week up to a target time point to take into account adaptation of the human body to the air temperature.

The heat-stress effect estimating device (10) may include a room temperature measurement unit (13) that measures an ambient temperature of a subject, for example, a room temperature in a room where the subject is present. The heat-stress effect estimating device (10) may further include, in addition to the room temperature measurement unit (13), a function of measuring or inputting a humidity, a radiant heat, an air flow, or the like around the subject, or a metabolic rate, an amount of clothing, or the like of the subject. The heat-stress effect estimating device (10) may also include an outdoor air temperature measurement unit (14) that measures an outdoor air temperature. The heat-stress effect estimating device (10) may also include an environment information storage unit (15) that stores the room temperature measured by the room temperature measurement unit (13) and the outdoor air temperature measured by the outdoor air temperature measurement unit (14). The heat-stress effect estimating device (10) may also include a state estimation target period determination unit (16) that determines a target period (such as one month or one season, for example) in which the effect of the heat stress is to be estimated. When determining the target period, the state estimation target period determination unit (16) may set, as the target, only a specific time period (for example, a period of business hours) among 24 hours of the day.

The configuration described above enables the calculation unit (12) to use the room temperature and the outdoor air temperature stored in the environment information storage unit (15) to estimate a state of the subject in terms of the index in the predetermined period determined by the state estimation target period determination unit (16). In this case, the calculation unit (12) may calculate an average room temperature and an average outdoor air temperature of each day by using the room temperatures and the outdoor air temperatures stored in the environment information storage unit (15) and may store the calculation results in the environment information storage unit (15). The calculation unit (12) may use an operation history (a history of the set temperature) of an air conditioner, instead of the room temperatures stored in the environment information storage unit (15). The calculation unit (12) may use outdoor air temperature information acquired from Internet such as from the AMeDAS, instead of the outdoor air temperatures stored in the environment information storage unit (15).

The calculation unit (12) may estimate the state of the subject in terms of the index over the predetermined period and may calculate a cumulative frequency of the estimated state.

The calculation unit (12) may classify the estimated state in accordance with at least one of a degree of the state, a duration of the state, and an occurrence time of the state, and may calculate a cumulative frequency for each classification or create a histogram.

The calculation unit (12) may compare the estimated state with a state of a person prepared in advance in terms of the index on a per-health-condition basis and may estimate a health condition of the subject.

The heat-stress effect estimating device (10) may include a display unit (17) that displays the state of the subject estimated in terms of the index by the calculation unit (12) and/or secondary information obtained based on the state (for example, frequencies of various states (an awake state, a sleepy state, and so on) of the subject).

In a heat-stress effect estimating method using the heat-stress effect estimating device (10), a heat stress reaction model ("an effect of an ambient environment on a person" = F(an ambient temperature, an outdoor air temperature)) is created in advance in which the effect of the ambient (room) environment on the person is associated with ambient temperature information indicating a state of the ambient environment and with outdoor air temperature information for use in correction of an error caused by temperature adaptation characteristics of the person. In addition, ambient temperature (room temperature) data and outdoor air temperature data corresponding to the ambient temperature data are collected. By using these pieces of data and the heat stress reaction model (for example, a sleepiness model), ambient temperature information (room temperature information) is converted into the effect of the heat stress on the subject. Further, the effect, that is, frequencies of the various states (the awake state, the sleepy state, and so on) of the subject are visualized. That is, a temperature history experienced by the subject is converted into the effect of the heat stress on the subject, and the effect is visualized.

In the heat-stress effect estimating device (10), the room temperature measurement unit (13) may be a temperature sensor carried by the subject or temperature sensors installed at a plurality of places (such as a room, a public facility, and outdoors). In the latter case, the ambient temperature of the subject is calculated based on temperature history data measured by the temperature sensors installed at the plurality of places and movement history data of the subject. The movement history data of the subject may be stored in advance or may be set and input by the subject appropriately or in accordance with a schedule of that day. Alternatively, the movement history data of the subject may be obtained, for example, by sensing, with an occupancy sensor installed in each room, the presence state of the subject in the room and accumulating presence information of the subject. Methods for sensing the presence state include a method for recognizing/determining the subject through image recognition, an automatic detection method using a location detection technique of a mobile phone or the like, and so on. The movement history data of the subject is associated with the accumulated temperature history data for the plurality of places. This enables the ambient temperature of the subject at the predetermined time point to be known.

Note that the heat-stress effect estimating device (10) includes a computer such as a microcomputer. The computer executes a program, so that each of the functions such as the calculation unit (12), that is, the heat-stress effect estimating method according to the present embodiment is carried out. The computer includes, as a main hardware component, a processor that operates in accordance with the program. The processor may be any kind of processor that can implement the functions by executing the program. For example, the processor may be constituted by, for example, one or a plurality of electronic circuits including a semiconductor integrated circuit (IC) or an LSI (large scale integration). The plurality of electronic circuits may be integrated into one chip, or may be disposed on or in a plurality of chips. The plurality of chips may be put together in one device or may be included in a plurality of devices. The program is recorded on a non-transitory recording medium such as a computer-readable ROM, optical disc, or hard disk drive. The program may be stored on the recording medium in advance, or may be supplied to the recording medium via a wide area communication network including Internet or the like.

As the heat stress reaction model storage unit (11) and the environment information storage unit (15), a computer-readable-writable recording medium, for example, a RAM or the like may be used. The heat stress reaction model storage unit (11) and the environment information storage unit (15) may be constituted by the same recording medium. As the state estimation target period determination unit (16), for example, a keyboard, a mouse, a touchpad, or the like may be used. As the display unit (17), for example, a monitor capable of displaying an image, such as a CRT display or a liquid crystal display may be used.

A manner in which the heat-stress effect estimating device (10) is implemented is not limited but the heat-stress effect estimating device (10) may be implemented in a remote control of an air conditioner, for example. In this case, the heat stress reaction model storage unit (11), the calculation unit (12), the display unit (17), and the like may be constituted by a microcomputer, a memory, a touch panel, and so on of the remote control.

### <Use of Estimation Result of Effect of Heat Stress>

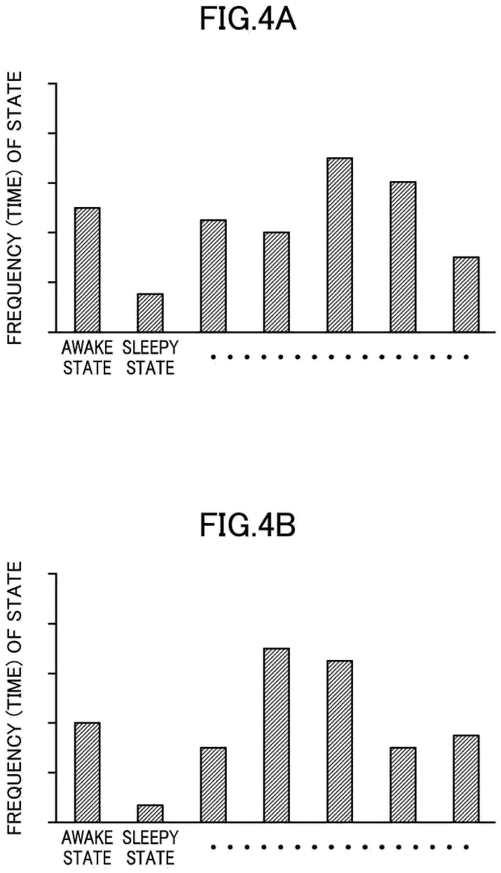

Each of Figs. 4A and 4B is a histogram of an example of frequencies (cumulative times) of the respective states of a subject having experienced a different temperature history obtained in terms of various indices by the heat-stress effect estimating device (10) according to the present embodiment. As illustrated in Figs. 4A and 4B, if the temperature histories experienced by the subject are different, patterns of the histograms are also different.

The states of the subject in terms of the indices used in the histograms illustrated in Figs. 4A and 4B are not limited. For example, "awaking", "getting sleepy", "body relaxing", "body tiring", "reaction time increasing", "reaction time decreasing", "incorrect answers decreasing", "incorrect answers increasing", "sympathetic nerve becoming dominant", "parasympathetic nerve becoming dominant", "skin moisturizing", "skin drying", and so on may be used.

Figs. 4A and 4B represent the state of (the effect of the heat stress on) the subject in terms of the indices as histograms. However, a manner in which the effect of the heat stress is represented is not limited. As information representing the effect (accumulated amount) of the heat stress to which the subject is exposed over a predetermined period, for example, a cumulative value or a statistical value (such as an average value, a variance, a maximum value, a minimum value, or a median value) may be calculated for each of the indices or a frequency with which (cumulative time for which) a state value of a predetermined value or greater is continued may be calculated for each of the indices, based on the state of the subject estimated in terms of the indices by the calculation unit (12), and the calculation result may be displayed on the display unit (17) as numerical values or a graph.

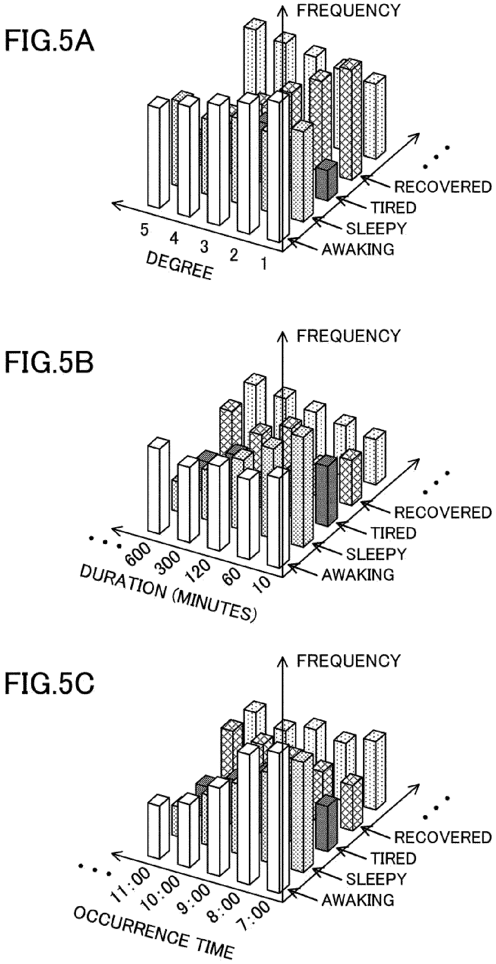

Figs. 5A, 5B, and 5C are diagrams illustrating an example of a result obtained by the heat-stress effect estimating device (10) according to the present embodiment by classifying the states of the subject obtained in terms of the various indices in accordance with degrees of the states (strength or weakness with respect to a criterion), durations of the states, and occurrence times of the states, respectively, and by calculating cumulative frequencies for the respective classifications. The states of the subject in terms of the indices are visualized separately for the "degrees", the "durations", and the "occurrence times" as illustrated in Figs. 5A, 5B, and 5C, respectively. This enables the effect of the heat stress to be evaluated from multiple perspectives.

Note that the manner of classification according to the "degrees", the "durations", or "the occurrence times" is not limited. As for the "degrees", the classification may be performed by strength-weakness-indicating levels from 1 to 10, for example. As for the "durations", the classification may be performed by 10 minutes, 30 minutes, 1 hour, 5 hours, and so on, for example. As for the "occurrence times", the classification may be performed by morning, afternoon, and evening or may be by time periods such as 7:00-9:00, for example.

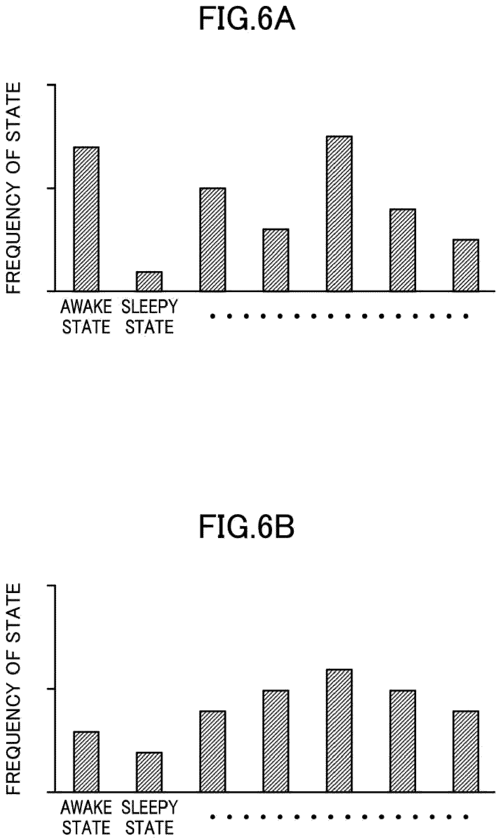

Figs. 6A and 6B are diagrams illustrating an example of states of a "healthy person" and a "person in a presymptomatic state due to an environment" in terms of the indices, used in the heat-stress effect estimating device (10) according to the present embodiment. The "presymptomatic state due to an environment" refers to a poor physical condition due to an environment, such as a cold sensitivity or a decrease in autonomic function due to a temperature difference, for example. If the states of a person in terms of the indices are prepared in advance on a per-health-condition basis as illustrated in Figs. 6A and 6B, a health condition of the subject can be estimated by comparing these states of the person in terms of the indices on a per-health-condition basis with the states of the subject estimated in terms of the indices.

That is, an association between "frequency patterns of states of a person estimated in terms of various indices" and "a health condition of the person" is converted in advance into data such as a histogram of a healthy person (see Fig. 6A) and a histogram of a person in a presymptomatic state (see Fig. 6B), for example. By comparing the histograms with the histogram (see, for example, Fig. 4A or 4B) of the subject, whether the subject is healthy or presymptomatic (for example, whether the subject has a disturbance in the autonomic nervous system because of excessive cooling) can be estimated.

Histograms are not necessarily used in the above-described comparison of the state estimated in terms of the indices. That is, by comparing the history of each state of the subject in terms of the appropriately selected index with the histories of each state of the healthy person and the person in a presymptomatic state, the health condition of the subject can be determined.

As described above, if health information related to a thermal environment can be acquired, the diagnosis accuracy of an environment-related presymptomatic state such as, for example, a cold sensitivity can be increased by adding the health information to a conventional medical examination report.

If health information related to the thermal environment can be acquired, the health information can be used as basis data in a consulting service or the like for giving advice on the good way of use of the air conditioner.

### -Advantages of Embodiment-

A heat-stress effect estimating device (10) according to the present embodiment includes a storage unit (11) configured to store a correlation of at least an ambient temperature of a person and an outdoor air temperature with at least one index indicating an effect of a heat stress on a person, and a calculation unit (12) configured to estimate a state of a subject in terms of the at least one index, based on the correlation stored in the storage unit (11), an ambient temperature of the subject at a predetermined time point, and a history of an outdoor air temperature up to the predetermined time point. Thus, a correlation of an ambient temperature of a person and an outdoor air temperature with an index indicating an effect of a heat stress on a person is determined in advance. Based on the correlation, an ambient temperature of a subject, and a history of an outdoor air temperature, the state of the subject can be estimated in terms of the index. Thus, the effect of the heat stress can be easily estimated not only for a reaction caused in a person by the heat stress such as a likelihood of heatstroke but also for various reactions caused in the person by the heat stress such as wakefulness and fatigue through selectin of the index. Consequently, since it becomes easier to grasp the effect of the accumulated heat stress on health, for example, the effect of the accumulated heat stress can be made use of in prevention of the occurrence of a disease.

However, simply determining or visualizing frequencies of the ambient temperature of the subject, for example, the room temperature is not enough to use the frequencies in evaluation of an effect on health or the like because even at the same room temperature, the state of the subject or a suitable situation (scene) changes in accordance with the outdoor air temperature.

In contrast, the heat-stress effect estimating device (10) according to the present embodiment estimates various states (such as an awake state and a sleepy state, for example) of the subject from room temperature data measured by a room temperature sensor and outdoor air temperature data at that time by using a relational model formula (for example, a relational model formula of a boundary temperature of sleepiness) that represents an effect of a room environment on a person based on, for example, room temperature information that represents a state of the room environment and average air temperature information for use in correction of an error caused by temperature adaptation characteristics of the person. The heat-stress effect estimating device (10) according to the present embodiment visualizes the cumulative times or the like of the respective states in a predetermined period. For example, the relational model formula of the boundary temperature of sleepiness is obtained by converting, into a numerical formula, a relation between a room temperature serving as the boundary of sleepiness and an outdoor air temperature, based on a database acquired by performing tests on subjects under various temperature-humidity environment conditions.

If, for example, a graph that visualizes cumulative times of the respective states of the subject is created by using the heat-stress effect estimating device (10) according to the present embodiment, the health condition of the subject can be estimated by comparing the graph with a graph of a person having each health condition (such as a graph of a healthy person or a graph of a person in a presymptomatic state) created in advance based on the database. That is, whether the subject has been exposed to an unbalanced thermal environment, for example, whether the subject has been in an environment in which the sympathetic nerve is always dominant and the time when the subject gets sleepy is short or in an environment in which the time when the sympathetic nerve is dominant and the time when the parasympathetic nerve is dominant are well balanced can be easily checked.

If the heat-stress effect estimating device (10) according to the present embodiment is used, an environment-related health condition is known from, for example, room temperature information for a room environment where the subject is present or a history of a set temperature of an air conditioner. Thus, the room environment can be improved for the health of the subject. Whether the room environment is suitable for the purpose, for example, whether the room environment is a thermal environment for awaking and improving the workability in the daytime or whether the room environment is a thermal environment in which the parasympathetic nerve is dominant and sleepiness is slightly induced in the nighttime can be easily checked.

Note that the heat-stress effect estimating device (10) according to the present embodiment can more accurately estimate the effect of the heat stress if a converted temperature determined by taking into account at least one of an ambient humidity, an ambient radiant heat, an ambient air flow, a metabolic rate, and an amount of clothing of the subject is used as the ambient temperature of the subject.

The heat-stress effect estimating device (10) according to the present embodiment can also estimate the effect of the heat stress in terms of a degree of wakefulness or a degree of fatigue if the degree of wakefulness or the degree of fatigue is used as the index indicating the effect of the heat stress on the person.

The heat-stress effect estimating device (10) according to the present embodiment can also evaluate an amount of heat stress accumulated over a predetermined period if the state of the subject is estimated in terms of the index over the predetermined period and a cumulative frequency of the estimated state is calculated with the calculation unit (12) .

The heat-stress effect estimating device (10) according to the present embodiment can also evaluate the effect of the heat stress from multiple perspectives if the estimated state is classified in accordance with at least one of the degree of the state, the duration of the state, and the occurrence time of the state and the cumulative frequency is calculated for each classification with the calculation unit (12) .

The heat-stress effect estimating device (10) according to the present embodiment can easily measure the ambient temperature of the subject also when the subject moves if the ambient temperature of the subject is measured by a temperature sensor carried by the subject.

The heat-stress effect estimating device (10) according to the present embodiment can measure the ambient temperature of the subject also when the subject moves without carrying a temperature sensor if the ambient temperature of the subject is calculated based on temperature information measured by temperature sensors installed at a plurality of places and a movement history of the subject.

The heat-stress effect estimating device (10) according to the present embodiment can evaluate the effect of the heat stress in an easy-to-understand manner if the heat-stress effect estimating device (10) according to the present embodiment further includes a display unit (17) that displays the estimated state and/or secondary information obtained based on the state.

It becomes easier for the heat-stress effect estimating device (10) according to the present embodiment to accurately grasp the effect of the heat stress on health if the health condition of the subject is estimated by comparing the estimated state with a state of a person prepared in advance in terms of the index on a per-health-condition basis with the calculation unit (12). Thus, the estimated state can be made use of in prevention of the occurrence of a disease.

### <Modification>

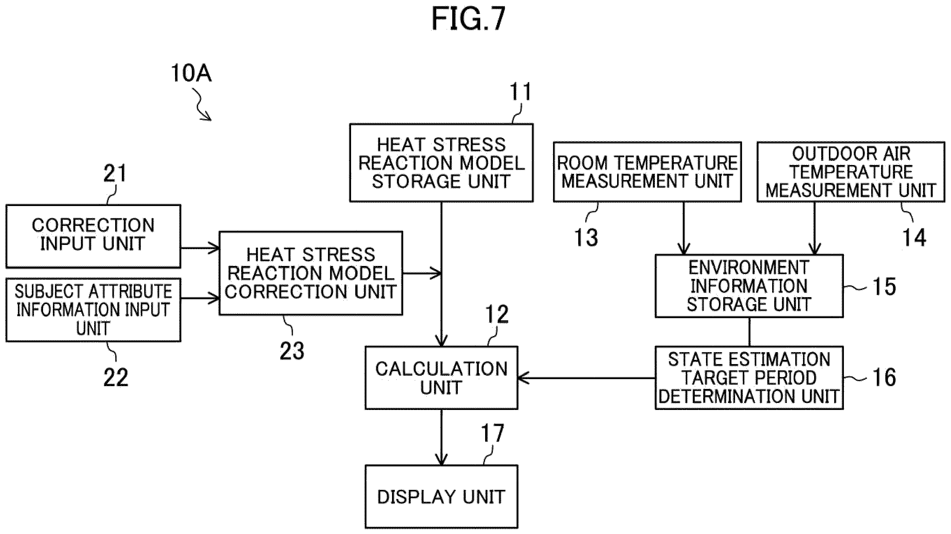

Fig. 7 is a block configuration diagram of a heat-stress effect estimating device according to a modification.

Differences of a heat-stress effect estimating device (10A) according to the present modification from the heat-stress effect estimating device (10) according to the embodiment illustrated in Fig. 3 are that a correction input unit (21), a subject attribute information input unit (22), and a heat stress reaction model correction unit (23) are further included as illustrated in Fig. 7 so that a correlation (heat stress reaction model) stored in a heat stress reaction model storage unit (11) can be corrected in accordance with an individual difference of a subject.

The correction input unit (21) is used by a subject to whom the heat stress reaction model is applied to input a setting for correcting the model in accordance with their preference. The subject can correct, for example, the boundary temperature through the correction input unit (21).

The subject attribute information input unit (22) is used by the subject to whom the heat stress reaction model is applied to input their attribute information. The subject can input, for example, their gender, age, metabolic rate, body fat percentage, blood pressure, or the like through the subject attribute information input unit (22).

As the correction input unit (21) and the subject attribute information input unit (22), for example, a keyboard, a mouse, a touchpad, or the like may be used.

The heat stress reaction model correction unit (23) corrects the heat stress reaction model stored in the heat stress reaction model storage unit (11), based on content input to the correction input unit (21) or the subject attribute information input unit (22). When the subject attribute information input unit (22) is used, a plurality of heat stress reaction models may be prepared for different pieces of attribute information and may be stored in the heat stress reaction model storage unit (11) in advance, and from among the plurality of heat stress reaction models, the heat stress reaction model correction unit (23) may select one model in accordance with the content input to the subject attribute information input unit (22). Note that the heat stress reaction model correction unit (23) may be configured to be integral with the calculation unit (12).

### -Advantages of Modification-

The heat-stress effect estimating device (10A) according to the present modification can correct the heat stress reaction model (correlation) stored in the storage unit (11), based on a setting input by the subject or the attribute information of the subject by using the correction input unit (21), the subject attribute information input unit (22), and the heat stress reaction model correction unit (23). Thus, the effect of the heat stress can be estimated by taking into account a difference between individuals.

### «Other Embodiments»

In the embodiment (including the modification) described above, the state of the subject is estimated in terms of the index using the heat stress reaction model, based on the ambient temperature of the subject and the history of the outdoor air temperature. To remove a factor not related to a thermal environment such as a psychological factor from history information of the estimated state, the subject may be caused to carry a sensor for measuring sweating, pulse waves, an electrocardiogram, or the like to acquire physiological information.

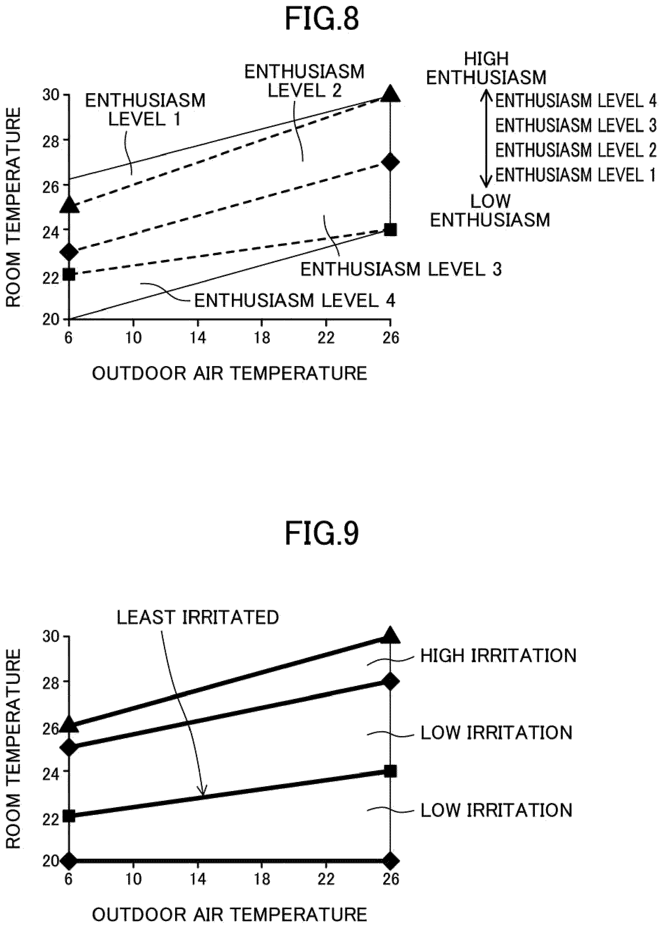

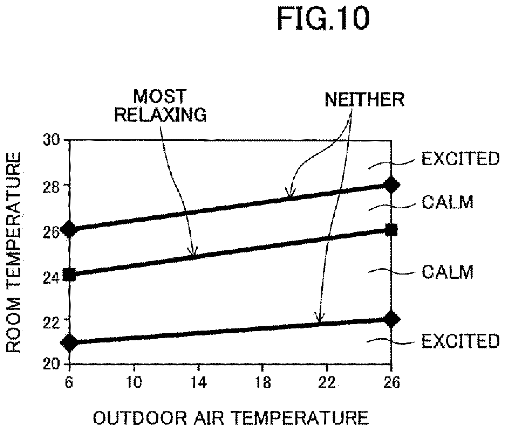

In the embodiment (including the modification) described above, the heat stress reaction model is created by using sleepiness (a degree of wakefulness) or fatigue (a degree of fatigue) as the effect of the heat stress caused by the ambient temperature on a person. However, the effect of the heat stress caused by the ambient temperature on a person is not limited, and the heat stress reaction model may be created by using, for example, a degree of enthusiasm (motivation), a degree of irritation, a degree of relaxing, or the like in addition to the degree of wakefulness and the degree of fatigue. Fig. 8 is a diagram illustrating an example of a correlation of an ambient temperature of a person and an outdoor air temperature with a degree of enthusiasm, which has been found by the inventors of this application. Fig. 9 is a diagram illustrating an example of a correlation of an ambient temperature of a person and an outdoor air temperature with a degree of irritation, which has been found by the inventors of this application. Fig. 10 is a diagram illustrating an example of a correlation of an ambient temperature of a person and an outdoor air temperature with a degree of relaxing, which has been found by the inventors of this application.

While the embodiment and modification have been described above, it should be understood that various modifications can be made on the configurations and details without departing from the gist and the scope of the claims. The embodiment, the modification, and the other embodiments described above may be combined or replaced as appropriate as long as the functionality of the target of the present disclosure is not reduced.

### Industrial Applicability

The present disclosure is useful for a heat-stress effect estimating device and a heat-stress effect estimating method.

### Reference Signs List

- 10, 10A: heat-stress effect estimating device
- 11: heat stress reaction model storage unit
- 12: calculation unit
- 13: room temperature measurement unit
- 14: outdoor air temperature measurement unit

- 15: environment information storage unit
- 16: state estimation target period determination unit
- 17: display unit
- 21: correction input unit
- 22: subject attribute information input unit
- 23: heat stress reaction model correction unit

## Claims

1. A heat-stress effect estimating device for estimating an effect of a heat stress imposed on a subject by an ambient temperature of the subject, comprising:
a storage unit (11) configured to store a correlation of at least an ambient temperature of a person and an outdoor air temperature with at least one index indicating an effect of a heat stress on a person; and
a calculation unit (12) configured to estimate a state of the subject in terms of the at least one index, based on the correlation stored in the storage unit (11), an ambient temperature of the subject at a predetermined time point, and a history of an outdoor air temperature up to the predetermined time point.

2. The heat-stress effect estimating device according to Claim 1, wherein
a converted temperature determined by taking into account at least one of an ambient humidity, an ambient radiant heat, an ambient air flow, a metabolic rate, and an amount of clothing of the subject is used as the ambient temperature of the subject.

3. The heat-stress effect estimating device according to Claim 1 or 2, wherein
the at least one index is at least one of a degree of wakefulness, a degree of fatigue, a degree of enthusiasm, a degree of irritation, and a degree of relaxing.

4. The heat-stress effect estimating device according to any one of Claims 1 to 3, wherein
the calculation unit (12) estimates the state over a predetermined period and calculates a cumulative frequency of the estimated state.

5. The heat-stress effect estimating device according to any one of Claims 1 to 4, wherein
the calculation unit (12) classifies the estimated state in accordance with at least one of a degree of the state, a duration of the state, and an occurrence time of the state, and calculates a cumulative frequency for each classification.

6. The heat-stress effect estimating device according to any one of Claims 1 to 5, further comprising:
a correction unit (23) configured to correct the correlation stored in the storage unit (11), based on a setting input by the subject or attribute information of the subject.

7. The heat-stress effect estimating device according to any one of Claims 1 to 6, wherein
the ambient temperature of the subject is measured by a temperature sensor carried by the subject.

8. The heat-stress effect estimating device according to any one of Claims 1 to 6, wherein
the ambient temperature of the subject is calculated based on temperature information measured by temperature sensors installed at a plurality of places and a movement history of the subject.

9. The heat-stress effect estimating device according to any one of Claims 1 to 8, further comprising:
a display unit (17) configured to display the estimated state and/or secondary information obtained based on the state.

10. The heat-stress effect estimating device according to any one of Claims 1 to 9, wherein
the calculation unit (12) compares the estimated state with a state of a person prepared in advance in terms of the at least one index on a per-health-condition basis and estimates a health condition of the subject.

11. A heat-stress effect estimating method for estimating an effect of a heat stress imposed on a subject by an ambient temperature of the subject, comprising:
a step A of, with a computer, determining a correlation of at least an ambient temperature of a person and an outdoor air temperature with at least one index indicating an effect of a heat stress on a person and storing the correlation in a storage unit (11); and
a step B of, with the computer, estimating a state of the subject in terms of the at least one index, based on the correlation stored in the storage unit (11), an ambient temperature of the subject at a predetermined time point, and a history of an outdoor air temperature up to the predetermined time point.

12. A computer program for causing a computer to perform the heat-stress effect estimating method according to Claim 11.
